# EUROPEAN PATENT SPECIFICATION

(11) **EP 0 772 975 B1**
(45) Date of publication and mention of the grant of the patent: **02.01.2002**
(21) Application number: 96116824.2
(22) Date of filing: 19.10.1996
(51) Int. Cl.: A01N 59/06, C01B 25/32

(54) **Microbes-removing material**
Material zur Entfernung von Mikroben
Matière pour l'élimination de microbes

(30) Priority: 13.11.1995 JP 29398495
(43) Date of publication of application: 14.05.1997
(73) Proprietor: MITSUBISHI MATERIALS CORPORATION, Chiyoda-ku, Tokyo (JP)
(72) Inventor: Okamoto, Kengo, Omiya-shi, Saitama-ken (JP)
(74) Representative: Gille Hrabal Struck Neidlein Prop Roos

(56) References cited:
- US-A- 5 227 147
- US-A- 5 427 754
- M. YOSHIMURA, H. SUDA, K. OKAMOTO & K. IOKU: "Hydrothermal synthesis of biocompatible whiskers" J. MATERIALS SCIENCE, vol. 29, 1994, pages 3399-3402, XP002046119
- PATENT ABSTRACTS OF JAPAN vol. 095, no. 009, 31 October 1995 & JP 07 146281 A (MITSUBISHI MATERIALS CORP), 6 June 1995,

## Description

### FIELD OF THE INVENTION

The present invention relates to a novel use of hydroxyapatite having a c-axis length of 1 to 1000 µm and an aspect ratio of 5 or more.

### BACKGROUND OF THE INVENTION

As microbes-removing materials and filters to be used in the food fermentation industry, in the cultivation and production of biochemical substances, in the production of artificial body fluids for medical use, and in the production of germ-free drinking water, there have heretofore been known inorganic porous substances, such as silica, activated charcoal and diatomaceous earth; non-woven fabrics or hollow fibers of organic fibers such as celluloses, polyethylenes and polyesters (see Japanese Patent Application Laid-Open Nos. 4-156933 and 6-63132); and fibrous metal substances of stainless steel, inconel, hastelloy or the like (see Japanese Patent Application Laid-Open No. 5-130858).

In addition, also employed as such microbes-removing materials are porous hydroxyapatite to be obtained from the hard tissue of vertebrates, and hydroxyapatite to be produced by wet processes.

Calcined hydroxyapatite to be prepared by calcining the above-mentioned hydroxyapatite at a temperature falling between 600°C and 1400°C is also employed as a microbes-removing material.

Conventional microbes-removing materials such as those mentioned above, for example, inorganic substances such as silica, activated charcoal and diatomaceous earth, as being porous, capture microbes in their pores to thereby remove them from the other substances.

Accordingly, such porous, inorganic substances can adsorb microbes of such sizes that can be captured in the pores of the inorganic substances but cannot adsorb the other microbes, which therefore shall pass through the inorganic substances together with the processed liquids.

In addition, the adsorption of microbes onto such porous, inorganic substances is not firm, as being mere physical bonding therebetween. Moreover, the adsorption capacity of the porous inorganic substances is small. For these reasons, the porous inorganic substances are not satisfactory for the removal of microbes to purify liquids.

On the other hand, in fibrous filters made of organic polymer fibers of celluloses, polyethylenes, polyesters or the like, or made of stainless steel, inconel, hastelloy or the like, the meshes of the fibrous materials are utilized as sieves. Therefore, such fibrous filters can merely physically fractionate and filter substances of different sizes.

The microbes-separating mechanism of hydroxyapatite is to attain ionizing adsorption, and is different from the physical adsorption to be attained by the above-mentioned, conventional microbes-separating materials. Therefore, hydroxyapatite has strong adsorption and separation characteristics specific to particular microbes. In addition, due to the stereospecific adsorbability of the adsorbing sites on its surface, hydroxyapatite is sensitive to the difference in the functional groups existing on the surfaces of microbes. For these reasons, hydroxyapatite is expected to have good adsorption and separation characteristics specific to the type of microbes.

However, hydroxyapatite that has heretofore been used in conventional microbes-separating materials is extracted from natural organisms or is synthesized by wet processes. Hydroxyapatite of this type is poorly crystalline and has poor adsorbability. In addition, it is in the form of fine particles of submicrons. Therefore, it cannot be shaped into filters by itself. If it is used as a microbes-removing material, its liquid permeability could not be ensured.

Calcined hydroxyapatite prepared by calcining wet-process synthesized hydroxyapatite at a temperature falling between 600°C and 1400°C may be used as a microbes-removing material. However, this could not satisfactorily adsorb acidic biochemical substances, namely, acidic microbes, since the growth of the A-plane of each particle crystal structure was not good.

The preparation of hydroxyapatite whiskers is described in US-A-5,227,147. The preparation includes heating by hydrothermal treatment and leads to hydroxyapatite whiskers with an average aspect ratio of 10.0 or higher and a c-axis length of from 1 to 1000 µm. The material described in the prior art document was used as a packed stationary phase in a column having a superior cell separation or cultivating efficiency, as a biotechnological material, such as carrier for cell cultivation or separation, as a variety of fiber-reinforced composite materials, or as a substitute construction material for asbestos without causing harmful effects on living organisms.

### SUMMARY OF THE INVENTION

Given the situation, we, the present inventors have assiduously studied in order to solve the above-mentioned problems and, as a result, have achieved the present invention which will be described in detail hereinunder.

According to the present invention, there is provided a method to remove microbes comprising using hydroxyapatite having a mean c-axis length of from 1 µm to 1000 µm and an aspect ratio of 5 or more.

Preferably, the method of the invention comprises using hydroxyapatite having a mean c-axis length of from 5 µm to 200 µm and an aspect ratio of 10 or more.

The invention relates to the use of the above-mentioned hydroxyapatite for the removal of microbes from a solution.

The present invention relates to removing bacteria and viruses in the food fermentation industry and in the cultivation and production of biochemical substances, and also removing bacteria and viruses from body fluids for medical use and from drinking water.

### DETAILED DESCRIPTION OF THE INVENTION

While being synthesized by hydrothermal reaction, the hydroxyapatite whiskers to be provided herein to be used as the microbes-removing material according to the use of the invention have good crystallinity and liquid-permeability. In addition, since the structure of the adsorbing sites on the surfaces of the crystals is well ordered, the crystalline hydroxyapatite whiskers have high adsorbability with large adsorption capacity and have good adsorption and separation characteristics.

Moreover, since the invention uses hydroxyapatite whiskers, of which the A-planes have been predominantly grown in the c-axis direction, these can remove not only microbes that shall be adsorbed by the whiskers on their C-planes but also those that shall be selectively adsorbed by them onto their A-planes.

Accordingly, the microbes-removing material used according to the present invention is sensitive to the difference in the functional groups existing on the surfaces of microbes, and therefore can selectively remove different kinds of microbes.

The ability of the microbes-removing material to selectively remove specific microbes may be for the following reasons: The hydroxyapatite, on which the present inventive use is based, is in the form of hexagonal pillar crystals having crystal planes with different properties, the A-plane corresponding to the side plane and the C-plane corresponding to the bottom plane.

The A-plane has adsorbing sites (C-sites) composed of positively-charged calcium ions, while the C-plane has adsorbing sites (P-sites) composed of negatively-charged phosphato ions.

Therefore, acidic biochemical substances having many carboxyl groups and phosphato groups shall be adsorbed onto the C-sites, while basic biochemical substances having many amino groups shall be adsorbed onto the P-sites. Such selectivity ensures the selective removability of the microbes-removing material of the invention. In the present invention, the hydroxyapatite whiskers are used in the form of layers or after having been shaped into filters.

Using the hydroxyapatite, therefore, all microbes which are larger than the pores of the material can be removed as residues, irrespective of the kind of microbes, while the others which are smaller than the pores thereof are selectively adsorbed by hydroxyapatite whiskers onto their adsorbing sites depending on the properties of the functional groups of the microbes.

The microbes-removing use is specifically connected to hydroxyapatite having a mean c-axis length of from 1 µm to 1000 µm and an aspect ratio of 5 or more.

The reason why the mean c-axis length of hydroxyapatite constituting the microbes-removing material of the present invention is defined to fall between 1 µm and 1000 µm is because, if the mean c-axis length is smaller than 1 µm, the material cannot have good liquid permeability, but if it is larger than 1000 µm, the meshes of the material are too large with the result that almost all microbes pass through such large meshes. The reason why the aspect ratio of hydroxyapatite constituting the microbes-removing material of the present invention is defined to be 5 or more is in order to enlarge the adsorbability of hydroxyapatite whiskers on their A-planes.

Preferably, the microbes-removing material to be provided by the present invention comprises hydroxyapatite having a mean c-axis length of from 5 µm to 200 µm and an aspect ratio of 10 or more.

The reason why the mean c-axis length of hydroxyapatite used according to the present invention is defined to be preferably not smaller than 5 µm is in order to ensure the satisfactory liquid permeability of the material; while the reason why it is defined to be preferably not larger than 200 µm is in order to reduce the number of microbes that may *pass* through the large meshes due to the c-axis length larger than 200 µm. On the other hand, the reason why the aspect ratio of hydroxyapatite used according to the present invention is defined to be preferably 10 or more is in order to enlarge more the adsorbability of hydroxyapatite whiskers on their A-planes.

The microbes as referred to herein include not only so-called bacteria but also viruses, rickettsiae, chlamydiae, mycoplasmae, spirochaetae, fungi, etc.

As mentioned hereinabove, the microbes-removing material can be used according to the invention by itself or after having been formed into layers or filters, to remove microbes. If desired, the material can be supported by any of non-woven fabrics, cottonlike stuff of natural or synthetic organic fibers, cottonlike stuff of inorganic fibers, or filter cloth or paper made of such fibers.

Preferred embodiments of the present invention are illustrated hereinunder, with reference to the following examples.

### Example 1:

25 g of lactic acid and 6.89 g of 85 % H₃PO₄ were dissolved in 500 ml of water, and 7.37 g of Ca(OH)₂ was added thereto. The resulting solution was subjected to hydrothermal treatment at 165°C for 5 hours to prepare a synthetic hydroxyapatite.

The c-axis length and the aspect ratio of this hydroxyapatite were measured with an optical microscope, resulting in that the mean c-axis length was 21 µm and the mean aspect ratio was 18.

Test liquids each containing cells of Escherichia coli, Staphylococcus aureus, influenza virus, separately in an amount of 10⁶ cells/ml were prepared. 0.1 g of the powdery hydroxyapatite synthesized in the above was added to each test liquid of 4 ml, and fully stirred. These test liquids were left stationary for 10 minutes, and the number of the cells in the resulting supernatant of each test liquid was counted, from which the ability of the hydroxyapatite to adsorb the cells was determined.

As comparative examples, the following samples were tested in the same manner as above.
(1) 112 g of calcium hydroxide was suspended in 3 liters of water, and phosphoric acid was dropwise added thereto, while stirring, to attain a molar ratio, Ca/P of 1.67. Thus was prepared a hydroxyapatite slurry, which was then dried, using a spray drier, into particles having a mean particle size of 15 µm and an aspect ratio of about 2.0. The wet-process hydroxyapatite thus synthesized was used as one comparative sample.
(2) The wet-process hydroxyapatite prepared above was calcined at 900°C for 1 hour to prepare a calcined hydroxyapatite having a mean particle size of 12 µm and an aspect ratio of about 2.5. This was used as another comparative sample.
(3) A high-purity silica having a mean particle size of 12 µm was used as still another comparative sample.
(4) As a blank control, nothing was added to the test liquids.

The results for Escherichia coli are shown in Table 1; those for Staphylococcus aureus in Table 2; and those for influenza virus in Table 3.

From these results, it is known that the ability of the hydrothermal-process hydroxyapatite to adsorb the cells is the greatest.

**Table 1**

| Escherichia coli | |
|---|---|
| Sample Tested | Number of Cells (/ml) |
| Hydrothermal-process Hydroxyapatite | 5.0 x 10³ |
| Wet-process Hydroxyapatite | 3.0 x 10⁵ |
| Calcined Hydroxyapatite | 7.0 x 10⁴ |
| Silica | 3.0 x 10⁶ |
| Blank | 5.0 x 10⁶ |

**Table 2**

| Staphylococcus aureus | |
|---|---|
| Sample Tested | Number of Cells (/ml) |
| Hydrothermal-process Hydroxyapatite | 2.0 x 10⁴ |
| Wet-process Hydroxyapatite | 3.0 x 10⁶ |
| Calcined Hydroxyapatite | 3.0 x 10⁵ |
| Silica | 3.0 x 10⁶ |
| Blank | 6.0 x 10⁶ |

**Table 3**

| Influenza Virus | |
|---|---|
| Sample Tested | Number of Cells (/ml) |
| Hydrothermal-process Hydroxyapatite | 7.0 x 10³ |
| Wet-process Hydroxyapatite | 4.0 x 10⁵ |
| Calcined Hydroxyapatite | 8.0 x 10⁴ |
| Silica | 2.0 x 10⁶ |
| Blank | 7.0 x 10⁶ |

### Example 2:

Three samples which are the same hydrothermal-process hydroxyapatite and calcined hydroxyapatite as those used in Example 1, and a cellulose filter-stuff having a filter pore size of 1 µm were tested herein. Cylindrical containers having a diameter of 8 mm and a length of 100 mm were separately filled with 10 g of each sample to prepare microbes-removing containers.

A test liquid containing cells of lactic acid bacteria (mean size: 5 µm) , Escherichia coli (mean size: 2 µm), Staphylococcus aureus (mean *size:* 0.5 µm), mycoplasma (mean size: 0.2 µm) and influenza virus (mean size: 0.1 µm) in an amount of 10⁶ cells/ml each was prepared. 10 ml of the test liquid was introduced into each microbes-removing container, at a flow rate of 3 ml/min, and the number of the cells in the resulting filtrate that passed through each container was counted.

The data obtained are shown in Table 4.

Since all the filtrates contained neither cells of lactic acid bacteria nor cells of Escherichia coli, the data for these were omitted in Table 4.

As shown in Table 4, the cellulose filter sample removed the bacteria larger than the filter pore size but could not sufficiently remove the others smaller than the filter pore size.

As opposed to this, the hydroxyapatite effectively removed all the tested microbes.

**Table 4**

| Sample Tested | Staphylococcus aureus | Mycoplasma | Influenza Virus |
|---|---|---|---|
| Hydrothermal-process Hydroxyapatite | 1.0 x 10² | 6.0 x 10² | 2.4 x 10³ |
| Calcined Hydroxyapatite | 7.0 x 10³ | 2.0 x 10⁴ | 6.5 x 10⁴ |
| Cellulose Filter | 1.0 x 10⁶ | 1.0 x 10⁶ | 1.0 x 10⁶ |

As has been described in detail hereinabove, since the microbes-removing material comprises hydroxyapatite having a mean c-axis length of from 1 µm to 1000 µm and an aspect ratio of 5 or more, it has good liquid permeability, high microbes-adsorbing power and large adsorption capacity. In addition, as having a large area ratio of A-plane, the hydroxyapatite can efficiently adsorb and remove even acidic microbes.

While the invention has been described in detail and with reference to specific embodiments thereof, it will be apparent to one skilled in the art that various changes and modifications can be made therein without departing from the scope thereof.

## Claims

1. Use of hydroxyapatite produced by hydrothermal reaction with a c-axis length of from 1 µm to 1000 µm and an aspect ratio of 5 or more for the removal of microbes from a solution.

2. The use according to claim 1, wherein the hydroxyapatite has a mean c-axis length of 5 µm to 200 µm.

3. The use according to claim 1 or 2, wherein the aspect ratio is 10 or more.

4. The use according to any of claims 1 to 3 wherein the microbes are selected from the group consisting of bacteria, viruses, rickettsiae, chlamydiae, mycoplasmae, spirochaetae, fungi and mixtures thereof.

5. The use according to any of claims 1 to 4, wherein the microbes have a mean size of 0.1 µm to 2 µm.

6. The use according to any of claims 1 to 5 wherein the microbes are either basic or acidic.

7. The use according to claim 6, wherein the microbes have surface carboxyl or phosphate groups and are acidic.

8. The use according to claim 6, wherein the microbes have surface amino groups and are basic.

9. The use according to any of the previous claims, wherein the solution is passed through a filter comprising said hydroxyapatite.

## Patentansprüche

1. Verwendung von durch eine Hydrothermalreaktion hergestelltem Hydroxyapatit mit einer c-Achsenlänge von 1 µm bis 1000 µm und einem Seitenverhältnis von 5 oder mehr zur Entfernung von Mikroben aus einer Lösung.

2. Verwendung nach Anspruch 1, wobei der Hydroxyapatit eine mittlere c-Achsenlänge von 25 µm bis 200 µm aufweist.

3. Verwendung nach Anspruch 1 oder 2, wobei das Seitenverhältnis 10 oder größer ist.

4. Verwendung nach einem der Ansprüche 1 bis 3, wobei die Mikroben ausgewählt werden aus der aus Bakterien, Viren, Rickettsien, Chlamydien, Mycoplasmen, Spirochäten, Pilzen und Gemischen davon bestehenden Gruppe.

5. Verwendung nach einem der Ansprüche 1 bis 4, wobei die Mikroben eine mittlere Größe von 0,1 µm bis 2 µm aufweisen.

6. Verwendung nach einem der Ansprüche 1 bis 5, wobei die Mikroben entweder basisch oder sauer sind.

7. Verwendung nach Anspruch 6, wobei die Mikroben Oberflächen-Carboxyl- oder-Phosphatgruppen aufweisen und sauer sind.

8. Verwendung nach Anspruch 6, wobei die Mikroben Oberflächen-Aminogruppen aufweisen und basisch sind.

9. Verwendung nach einem der vorstehenden Ansprüche, wobei die Lösung durch einen den Hydroxyapatit umfassenden Filter gegeben werden.

## Revendications

1. Utilisation de l'hydroxyapatite produite par une réaction hydrothermale ayant une longueur d'axe c de 1 µm à 1 000 µm et un rapport d'aspect de 5 ou plus pour l'élimination de microbes d'une solution.

2. Utilisation selon la revendication 1, dans laquelle l'hydroxyapatite possède une longueur moyenne d'axe c de 5 µm à 200 µm.

3. Utilisation selon la revendication 1 ou 2, dans laquelle le rapport d'aspect est de 10 ou plus.

4. Utilisation selon l'une quelconque des revendications 1 à 3, dans laquelle les microbes sont choisis dans le groupe formé par les bactéries, les virus, les rickettsies, les chlamydies, les mycoplasmes, les spirochètes, les champignons et leurs mélanges.

5. Utilisation selon l'une quelconque des revendications 1 à 4, dans laquelle les microbes ont une taille moyenne de 0,1 µm à 2 µm.

6. Utilisation selon l'une quelconque des revendications 1 à 5, dans laquelle les microbes sont soit basiques soit acides.

7. Utilisation selon la revendication 6, dans laquelle les microbes possèdent des groupes phosphate ou carboxyle en surface et sont acides.

8. Utilisation selon la revendication 6, dans laquelle les microbes possèdent des groupes amino en surface et sont basiques.

9. Utilisation selon l'une quelconque des revendications précédentes, dans laquelle on fait passer la solution à travers un filtre comprenant ladite hydroxyapatite.
